# EUROPÄISCHE PATENTSCHRIFT

(11) **EP 0 349 862 B1**
(45) Veröffentlichungstag und Bekanntmachung des Hinweises auf die Patenterteilung: **27.10.1993**
(21) Anmeldenummer: 89111536.2
(22) Anmeldetag: 24.06.1989
(51) Int. Cl.: C07D 211/58, C07D 407/12, C07D 401/12, C08K 5/34

(54) **2,6-Polyalkyl-piperidin-4-amide, deren Verwendung als Stabilisatoren, insbesondere für Kunststoffe, sowie diese Amide enthaltendes organisches Material**
2,6 polyalkyl piperidine-4-amides, their use as stabilizers in particular for synthetic materials as well as the organic material containing them
2,6 polyalkyl pipéridine-4-amides, leur application comme stabilisateurs, en particulier pour matériaux synthétiques aussi bien que le matériau organique les contenant

(30) Priorität: 08.07.1988 DE 3823112
(43) Veröffentlichungstag der Anmeldung: 10.01.1990
(73) Patentinhaber: BASF Aktiengesellschaft, 67063 Ludwigshafen (DE)
(72) Erfinder: Aumueller, Alexander, Dr., D-6705 Deidesheim (DE); Neumann, Peter, Dr., D-6800 Mannheim 31 (DE); Trauth, Hubert, D-6724 Dudenhofen (DE)

(56) Entgegenhaltungen:
- EP-A- 0 117 225
- EP-A- 0 172 413

## Beschreibung

Es ist bekannt, daß Polyalkylpiperidinderivate organische Polymere vor Zerstörung durch Licht und Wärme schützen.

Solche Stabilisatoren sind z. B. aus der DE-OS 23 49 962 bekannt.

Im Dokument EP-A 117 225 werden als Stabilisatoren für Polymere geeignete Verbindungen beschrieben, welche zwei α, α, α', α' -tetrasubstituierte Piperidinteile durch eine Polyolkette verbindet, wobei die Verknüpfung dieser Polyolkette mit der 4-Position im Piperidinring jeweils über eine Carbamat-Funktion erfolgt und die Polyolkette keine weiteren stickstoffhaltigen Funktionen enthält.

Unbefriedigend ist bei den Stabilisatoren des Standes der Technik oft deren schlechte Verträglichkeit mit Polyolefinen und anderen Kunststoffen, die Dauer der Schutzwirkung, sowie deren Flüchtigkeit und Zersetzung beim Einarbeiten bei erhöhter Temperatur insbesondere in aggressiven Polymermedien.

Der Erfindung lag die Aufgabe zugrunde, neue Stabilisatoren zur Verfügung zu stellen, welche die vorstehenden Nachteile nicht aufweisen.

Die Aufgabe wird mit den neuen Polyalkylpiperidinamiden gelöst. Dementsprechend betrifft die Erfindung neue Verbindungen der allgemeinen Formel (I) 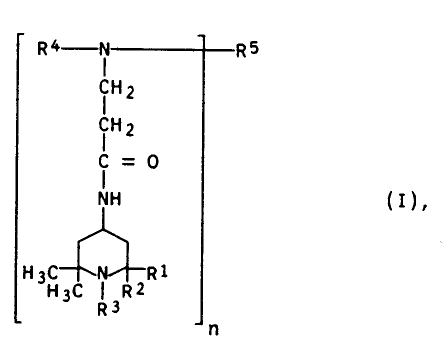
in der
- R¹ und R²: Methyl,
- R³: Wasserstoff, C₁- bis C₈-Alkyl, C₁- bis C₈-Alkoxycarbonyl, C₁-C₈-Alkanoyl, Benzyl, Hydroxyethyl, Cyanmethyl oder Aminoethyl und
- n: 1, 2 oder 3 bedeuten, und - wenn n = 1 ist - stehen
- R⁴: für Wasserstoff und
- R⁵: a) für 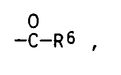 worin
- R⁶: Wasserstoff, C₁-C₁₉-Alkyl, C₇-C₁₀-Phenylalkyl, worin der Phenylkern gegebenenfalls durch 1, 2 oder 3 C₁-C₈-Alkyl, Chlor, Brom, Fluor, Hydroxy, C₁-C₄-Alkoxy, Phenyl, Phenoxy oder Carboxy substituiert ist und die Substituenten gleich oder verschieden sein können, Phenoxy-C₁-C₄-alkyl, gegebenenfalls durch 1 bis 3 C₁-C₈-Alkyl substituiertes C₃-C₁₀-Cycloalkyl; gegebenenfalls durch C₁-C₈-Alkyl, Chlor, Brom, Fluor, Hydroxy, C₁-C₄-Alkoxy, Phenyl, Phenoxy oder Carboxyl substituiertes Phenyl oder Naphthyl und worin die Zahl der Substituenten bis zu 3 betragen kann; einen 5- oder 6-gliedrigen gesättigten oder aromatischen O, S oder N enthaltenden Heterocyclus, der gegebenenfalls durch 1 bis 4 C₁-C₄-Alkyl substituiert ist; C₁-C₁₈-Alkoxy, worin die Alkylkette durch -O- unterbrochen sein kann; Phenoxy, Naphthoxy, gegebenenfalls durch 1 oder 2 C₁-C₄-Alkyl substituiertes C₅- oder C₆-Cycloalkoxy oder Phenylalkoxy mit insgesamt 7 bis 12 C-Atomen ist,
b) für -CO-NH-R⁷, worin R⁷ Wasserstoff, C₁-C₁₈-Alkyl, C₅- oder C₆-Cycloalkyl, Phenyl, das gegebenenfalls durch 1 bis 3 C₁-C₈-Alkyl, Chlor, Brom, Fluor oder C₁-C₄-Alkoxy substituiert ist, oder Phenyl-C₁-C₄-Alkyl ist oder
c) für -SO₂- R⁸, worin R⁸ C₁-C₁₈-Alkyl oder gegebenenfalls durch C₁-C₄-Alkyl oder Chlor substituiertes Phenyl ist, oder 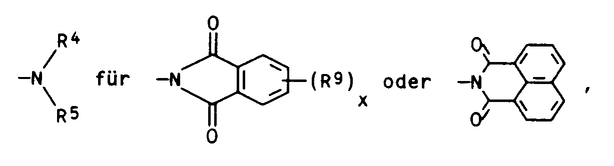 worin R⁹ C₁-C₈-Alkyl, Chlor, Brom oder Fluor und x = 0, 1, 2, 3 oder 4 bedeuten, oder - wenn n = 2 ist - stehen
- R⁴: für Wasserstoff und
- R⁵: für 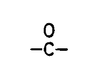 oder 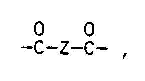 worin
- Z: eine chemische Bindung, C₁-C₁₂-Alkylen, C₄-C₈-Oxaalkylen, Phenylen, Cyclohexylen, Diphenylen, Diphenylenoxid, Dioxyphenylen, Diaminophenylen, Diaminocyclohexylen, 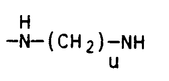 mit u = 2 bis 6, -O-X-O-, worin X C₂-C₈-Alkylen oder C₄-C₈-Oxaalkylen, oder ein zweiwertiger gesättigter oder ungesättigter 5- oder 6-gliedriger O, S oder N enthaltender Heterocyclus ist, oder 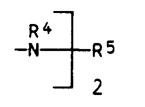 für eine Pyromellithsäurediimidgruppe,
oder - wenn n = 3 ist - stehen
- R⁴: für Wasserstoff und
- R⁵: 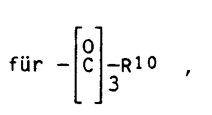 worin
- R¹⁰: einen dreiwertigen C₁-C₈-Alkylrest oder einen dreiwertigen Phenylrest bedeutet,
sowie Säureadditionssalze und Hydrate der Verbindungen (I).

Die erfindungsgemäßen Verbindungen (I) stabilisieren organisches Material, speziell Kunststoffe, gegen den Abbau durch Licht und Wärme. Sie sind auch wirksam als Metalldesaktivator. Den zu stabilisierenden Kunststoffen wird (I) in einer Konzentration von 0.01 bis 5 Gew.-% vorzugsweise von 0.02 bis 2 Gew.-% vor, während oder nach der Polymerbildung zugesetzt.

Die Polyalkylpiperidinamide (I) weisen im Polymeren gute stabilisierende Eigenschaften in Verbindung mit hervorragender Verträglichkeit auf. Besonders gute Eigenschaften zeigen die erfindungsgemäßen Verbindungen (I) in Polyolefinen, insbesondere in Ethylen- und Propylenpolymerisaten und in Polyamiden, außerdem in Polyurethanen und Lacken.

Alkylreste für R³ können linear oder verzweigt sein. Im einzelnen sind als Alkyl zu nennen: Methyl, Ethyl, iso-Propyl, Propyl, Butyl, iso-Butyl, Pentyl, Hexyl, Heptyl, Octyl, wovon Methyl bevorzugt ist. Alkyloxycarbonylgruppen für R³ sind beispielsweise Methoxycarbonyl, Ethoxycarbonyl, Propoxycarbonyl, Butoxycarbonyl, Pentoxycarbonyl, Hexoxycarbonyl, Heptoxycarbonyl und Octoxycarbonyl, wovon Methoxycarbonyl bevorzugt ist.

Acylgruppen für R³ sind C₁-C₈-Alkanoyl, z. B. Propionyl, Butanoyl, Pentanoyl, Hexanoyl, Heptanoyl oder Octanoyl, vorzugsweise Acetyl.
- R³: kann weiterhin auch Benzyl, Hydroxyethyl, Aminoethyl oder Cyanmethyl sein.

Besonders bevorzugt für R³ ist Wasserstoff.

Wenn n für 1 steht, kommen als Reste für R⁵ z.B. in Betracht: 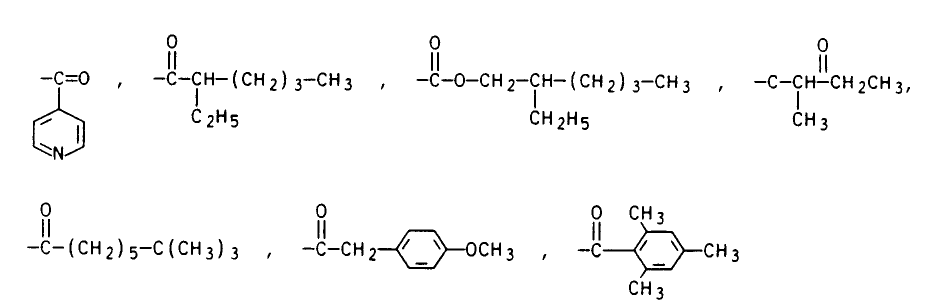 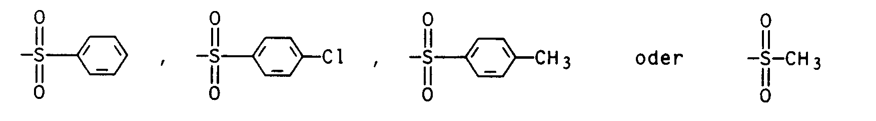
wobei
- x: 0 bis 20 und
- y: 0 bis 16 sind.

Wenn n = 1 ist, kann 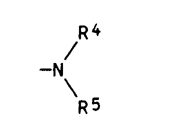
z.B. auch eine Gruppierung der Formeln sein: 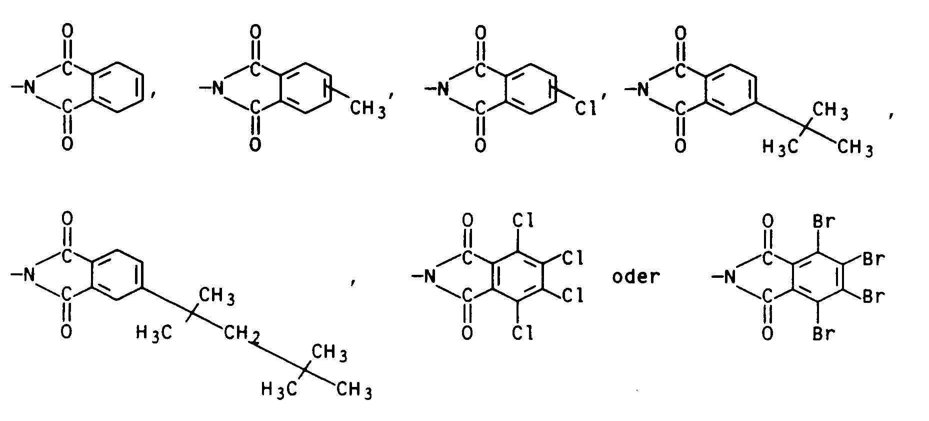
Wenn
- n: für 2 und
- R⁴: für Wasserstoff stehen, kann
- R⁵: beispielsweise sein: 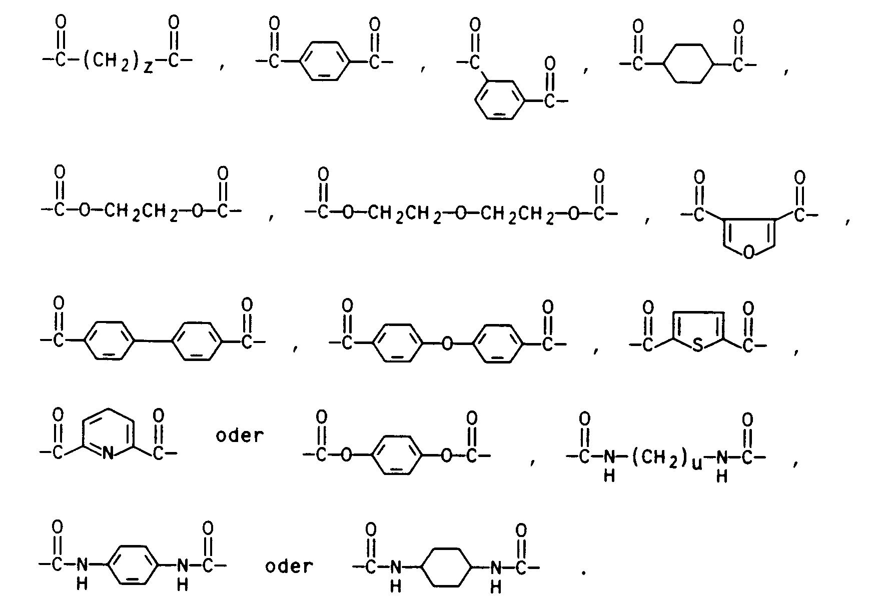 wobei
- z: 0 bis 12 und
- u: 2 bis 6 sein kann.

Die Reste R⁴ und R⁵ können beispielsweise zusammen auch eine Gruppierung der Formel 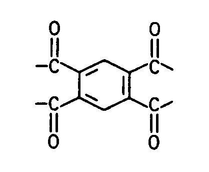
sein.

Wenn
- n: für 3 und
- R⁴: für Wasserstoff steht, kann
- R⁵: z. B. sein: 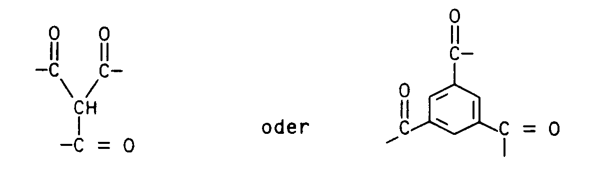 Die erfindungsgemäßen Verbindungen lassen sich beispielsweise durch Umsetzung von Verbindungen der allgemeinen Formel (II) 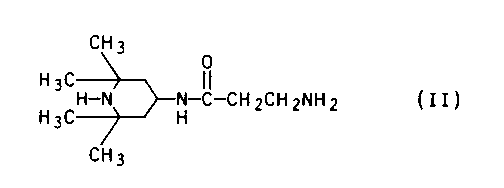 mit den R⁵ entsprechenden Carbonsäuren, Carbonsäureanhydriden, Carbonsäureestern, Sulfonsäurechloriden, Chlorformiaten, Harnstoffen oder Isocyanaten in an sich bekannter Weise herstellen.
Dabei kann eine Hilfsbase, z.B. ein tertiäres Amin wie Triethylamin oder Pyridin verwendet werden.
Vorzugsweise arbeitet man in inerten organischen Lösungsmitteln z.B. Dichlormethan, Chloroform, Tetrachlorkohlenstoff, Benzol, Toluol, Xylol, Acetonitril, Tetrahydrofuran oder Diethylether oder auch Essigsäureethylester oder Essigsäurebutylester.
Die Reaktion kann im Temperaturbereich von -20°C bis 100°C, vorzugsweise bei 0°C bis 25°C durchgeführt werden.

Verbindungen der allgemeinen Formel (I) mit R³ = H lassen sich nach literaturbekannten Verfahren, z.B. durch Acylierung, Alkylierung, Cyanmethylierung oder Ethoxylierung in Verbindungen der allgemeinen Formel (I) mit R³ ≠ H überführen.

Die erfindungsgemäßen Verbindungen können in Form der freien Basen, als Hydrate oder als Salze vorliegen. Geeignete Anionen stammen z. B. von anorganischen Säuren und insbesondere von organischen Carbonsäuren sowie von organischen Sulfonsäuren.

Anorganische Anionen sind z. B.: Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanoat, Cyclohexanoat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die durch die Verbindungen (I) stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel sowie Pigmente und Füllstoffe.

Die neuen Verbindungen (I) können nach bekannten Methoden und in den bekannten Vorrichtungen in die zu stabilisierenden Kunststoffe, gegebenenfalls mit weiteren Stabilisierungsmitteln und/oder anderen Zusätzen eingearbeitet werden.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel sind z. B. zu nennen: 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3′,5′-di-tert.-butyl-4′-hydroxy-benzyl)-benzol, 1,3,5-Tris-(3′,5′-di-tert.-butyl-4-hydroxybenzyl)-isocyanurat, 1,3,5-Tris-[β-(3′,5′-di-tert.-butyl-4′-hydroxyphenyl)-propionyloxy-ethyl]-isocyanurat, 1,3,5-Tris-(2′,6′-dimethyl-3′ -hydroxy-4′-tert.-butylbenzyl)-isocyanurat und Pentaerythrit-tetrakis-[β-(3,5-di-tert.-butyl-4-hydroxy-phenyl)-propionat].

Als phosphorhaltige Antioxidantien kommen z. B. in Betracht: Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4′-biphenylendiphosphit.

Als Schwefel enthaltende Antioxidationsmittel sind z. B. zu nennen: Dilaurylthiodipropionat, Dimyristylthiodipropionat, Distearylthio-dipropionat, Pentaerythrittetrakis-(β-laurylthioprioionat) und Penta-erythrittetrakis-(β-hexylthiopropionat).

Weitere Antioxidantien und Lichtstabilisatoren, die zusammen mit den erfindungsgemäßen Verbindungen (I) verwendet werden können, sind z. B. 2-(2′-Hydroxyphenyl)-benztriazole, 2-Hydroxybenzophenone, Arylester von Hydroxybenzoesäuren, α-Cyanozimtsäurederivate, Nickelverbindungen, Oxalsäuredianilide oder Benzimidazolcarbonsäureanilide.

Als organische Polymere, die mit den erfindungsgemäßen Verbindungen stabilisiert werden können, sind z. B. zu nennen:
Polymere von Mono- und Diolefinen, wie z. B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol;
Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methacrylat;
ABS-, MBS- oder ähnliche Polymere;
Halogenhaltige Polymere, wie z. B. Polyvinylchlorid, Polyvinylfluorid, Polyvinylidenfluorid sowie deren Copolymere;
Polymere die sich von α ·β-ungesättigten Säuren und deren Derivaten ableiten, wie Polyacrylate und Polymethacrylate, Polyacrylamide und Polyacrylnitrile;
Polymere, die sich von ungesättigten Alkoholen und Aminen bzw. deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weiterhin können Überzüge, die mit Hilfe von Lacken erzeugt worden sind, mit den Verbindungen (I) gegen den Abbau durch Licht und Wärme stabilisiert werden. Von diesen sind Einbrennlackierungen, insbesondere Fahrzeuglackierungen, vorzugsweise Zweischichtlackierungen, besonders hervorzuheben. Auch hier können zusätzlich die bereits genannten Antioxidantien und Lichtschutzmittel verwendet werden.

Die erfindungsgemäßen Verbindungen können den Lacken in fester oder gelöster Form zugesetzt werden. Ihre gute Löslichkeit in Lacksystemen ist dabei von besonderem Vorteil.

Die Erfindung soll durch die folgenden Beispiele zusätzlich weiter erläutert werden. Die Ausbeuten der Herstellbeispiele sind in keinem Fall optimiert.

### Herstellbeispiele

### Beispiel 1

800 ml Ethanol, 1356 g Cyanessigsäureethylester und 1860 g 2,2,6,6-Tetramethyl-4-aminopiperidin wurden 7 Stunden gekocht und anschließend im Eisbad auf 10 °C abgekühlt. Der ausgefallene Niederschlag wurde abgesaugt und mit kaltem Essigsäureethylester gewaschen. Man erhielt 1588 g der Verbindung der Formel 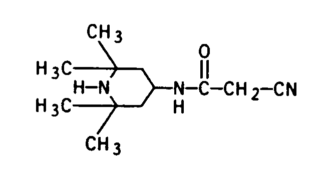
als farblosen Feststoff vom Schmp. 148°C.

### Beispiel 2

150 g des Produkts aus Beispiel 1 wurden unter Zusatz von 25 g Raney-Nickel und 100 g Ammoniak in 1000 ml Toluol bei 100°C und einem Wasserstoffdruck von 300 bar bis zur Druckkonstanz hydriert. Nach dem Abfiltrieren wurde eingeengt. Man erhielt 150 g der Verbindung der Formel 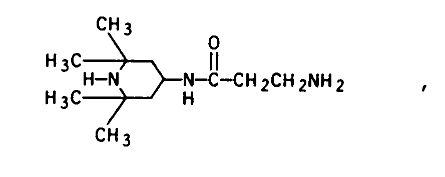
die nach längerem Stehen erstarrt und einen Schmelzpunkt von 59 - 60°C aufweist.

### Beispiel 3

34,0 g des Produktes aus Beispiel 2 und 15,2 g Triethylamin wurden in 150 ml Dichlormethan gelöst. Dazu tropfte man bei 0°C 11,8 g Acetylchlorid in 100 ml Dichlormethan und ließ auf Raumtemperatur kommen. Nach 5 Stunden Nachrühren wurde die Reaktionsmischung eingedampft, der Rückstand in Wasser gelöst und mit Natronlauge basisch gestellt. Die Wasserphase wurde mit n-Butanol extrahiert, die n-Butanolphase mit Wasser gewaschen und eingeengt. Der Rückstand wurde aus Acetonitril umkristallisiert. Man erhielt 26 g der Verbindung der Formel 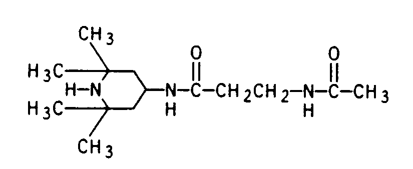
als farblosen Feststoff vom Schmp. 152°C

| | | | | | | | | |
|---|---|---|---|---|---|---|---|---|
| Ber. | C | 62,4 | H | 10,1 | N | 15,6 | O | 11,9 % |
| Gef. | C | 62,1 | H | 9,9 | N | 15,6 | O | 12,0 % |

### Beispiel 4

Zu 34 g des Produktes aus Beispiel 2 und 16,0 g Triethylamin in 200 ml Dichlormethan wurden bei 0 °C 13,9 g Propionsäurechlorid in 100 ml Dichlormethan getropft. Nach 3 Stunden bei Raumtemperatur wurden 20 g NaOH in 300 ml Wasser gelöst zugegeben. Die organische Phase wurde abgetrennt, über Magnesiumsulfat getrocknet und eingeengt. Der Rückstand wurde aus Toluol umkristallisiert, man erhielt 9,1 g der Verbindung der Formel 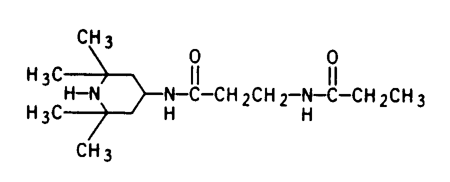
als farblosen Feststoff vom Schmp.: 149 - 153°C

| | | | | | | | | |
|---|---|---|---|---|---|---|---|---|
| Ber.: | C | 63,6 | H | 10,3 | N | 14,8 | O | 11,3 % |
| Gef.: | C | 62,7 | H | 10,3 | N | 14,5 | O | 12,3 % |

### Beispiel 5

34,0 g des Produktes aus Beispiel 2, 16,0 g Triethylamin und 16,0 g Buttersäurechlorid wurden wie in Beispiel 4 umgesetzt und aufgearbeitet. Umkristallisation aus Acetonitril lieferte 23,1 g der Verbindung der Formel 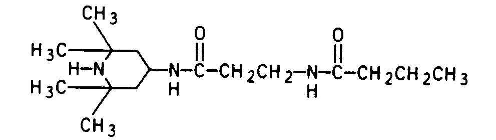
als farblosen Feststoff vom Schmp. 180 - 81°C.

| | | | | | | | | |
|---|---|---|---|---|---|---|---|---|
| Ber.: | C | 64,6 | H | 10,5 | N | 14,1 | O | 10,8 % |
| Gef.: | C | 64,5 | H | 10,4 | N | 14,1 | O | 10,7 % |

### Beispiel 6

Zu 34,0 g des Produktes aus Beispiel 2 und 16,0 g Triethylamin in 200 ml Dichlormethan wurden bei 0 °C 18,1 g Pivalinsäurechlorid in 100 ml Dichlormethan zugetropft. Nach 3 Stunden Rühren bei Raumtemperatur wurden 20 g Natronlauge, gelöst in 500 ml Wasser, zugegeben und die Phasen getrennt. Die wäßrige Phase wurde mit n-Butanol extrahiert, die organischen Phasen wurden vereinigt, über Magnesiumsulfat getrocknet, filtriert und eingeengt. Der Rückstand wurde aus Acetonitril umkristallisiert, man erhielt 25,9 g der Verbindung der Formel 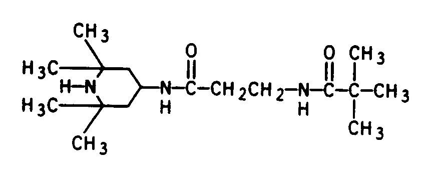
als farblosen Feststoff vom Schmp.: 177 - 178°C.

| | | | | | | | | |
|---|---|---|---|---|---|---|---|---|
| Ber.: | C | 65,6 | H | 10,7 | N | 13,5 | O | 10,3 % |
| Gef.: | C | 65,5 | H | 10,7 | N | 13,4 | O | 10,1 % |

### Beispiel 7

34,0 g des Produktes aus Beispiel 2, 16,0 g Triethylamin und 20,2 g tert.-Butylacetylchlorid wurden wie in Beispiel 4 umgesetzt und aufgearbeitet. Nach Umkristallisation aus Acetonitril erhielt man 24, 1 g der Verbindung der Formel 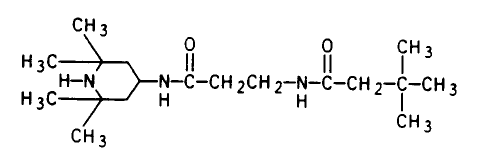
als farblosen Feststoff vom Schmp.: 148 - 151°C.

| | | | | | | | | |
|---|---|---|---|---|---|---|---|---|
| Ber.: | C | 66,4 | H | 10,8 | N | 12,9 | O | 9,8 % |
| Gef.: | C | 66,2 | H | 10,8 | N | 13,0 | O | 9,7 % |

### Beispiel 8

Zu 34,0 g des Produktes aus Beispiel 2 und 15,2 g Triethylamin in 400 ml Dichlormethan wurden bei 0 °C 24,4 g 2-Ethyl-1-hexansäurechlorid in 100 ml Dichlormethan zugetropft. Nach 4 Stunden bei Raumtemperatur wurde die Reaktionsmischung bis zur Trockne eingedampft, der Rückstand mit Wasser verrührt und mit Natronlauge alkalisch gestellt. Man saugte ab, wusch mit Wasser nach und kristallisierte den Rückstand aus Acetonitril um. Man erhielt 39 g der Verbindung der Formel 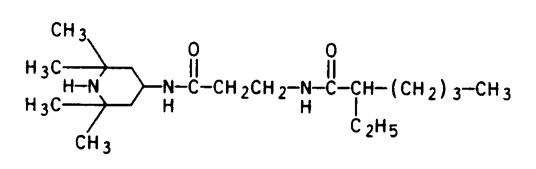
als farblosen Feststoff vom Schmp.: 192°C.

| | | | | | | | | |
|---|---|---|---|---|---|---|---|---|
| Ber.: | C | 67,9 | H | 11,1 | N | 11,9 | O | 9,0 % |
| Gef.: | C | 67,9 | H | 11,1 | N | 11,9 | O | 8,9 % |

### Beispiel 9

Zu 42,0 g des Produktes aus Beispiel 2 und 18,7 g Triethylamin in 150 ml Dichlormethan tropfte man bei 0 °C 26,0 g Benzoesäurechlorid in 100 ml Dichlormethan. Nach 2 Tagen bei Raumtemperatur wurden 200 ml Petrolether zugegeben, der ausgefallene Niederschlag wurde abgesaugt und in Wasser gelöst. Mit Natronlauge wurde alkalisch gestellt, die Mischung wurde mit n-Butanol extrahiert. Nach dem Abdampfen des n-Butanols wurde der Rückstand aus Acetonitril umkristallisiert. Man erhielt 50,0 g der Verbindung der Formel 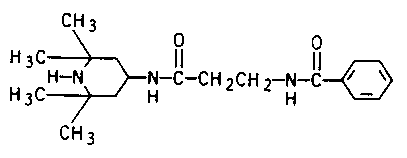
als farblosen Feststoff vom Schmp.: 190°C.

| | | | | | | | | |
|---|---|---|---|---|---|---|---|---|
| Ber.: | C | 68,8 | H | 8,8 | N | 12,7 | O | 9,6 % |
| Gef.: | C | 68,5 | H | 8,9 | N | 12,8 | O | 9,7 % |

### Beispiel 10

Zu 34,5 g des Produktes aus Beispiel 2 und 15,2 g Triethylamin in 200 ml Dichlormethan tropfte man bei 0 °C 27,4 g 2,4,6-Trimethylbenzoesäurechlorid in 75 ml Dichlormethan. Nach 16 Stunden Rühren bei Raumtemperatur wurde abgesaugt, mit Dichlormethan gewaschen und der Rückstand mit 250 ml Wasser verrührt. Man stellte mit Natronlauge alkalisch, saugte ab und wusch mit wenig Wasser nach. Umkristallisation des Rückstandes aus Acetonitril lieferte 12,0 g der Verbindung der Formel 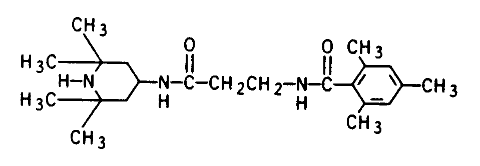
als farblosen Feststoff vom Schmp.: 229°C.

| | | | | | | | | |
|---|---|---|---|---|---|---|---|---|
| Ber.: | C | 70,7 | H | 9,4 | N | 11,2 | O | 8,6 % |
| Gef.: | C | 70,7 | H | 9,5 | N | 11,2 | O | 8,6 % |

### Beispiel 11

45,8 g des Produktes aus Beispiel 2 und 5,9 g Harnstoff wurden gemischt und 7 Stunden auf 130 - 140 °C erhitzt. Nach dem Abkühlen wurde aus Acetonitril umkristallisiert. Man erhielt 28,0 g der Verbindung der Formel 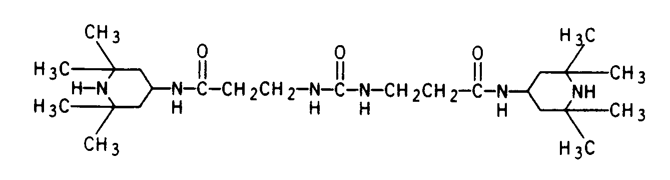
als farblosen Feststoff vom Schmp. : 217°C.

| | | | | | | | | |
|---|---|---|---|---|---|---|---|---|
| Ber.: | C | 62,5 | H | 10,1 | N | 17,5 | O | 10,0 % |
| Gef.: | C | 62,0 | H | 10,1 | N | 17,5 | O | 10,3 % |

### Beispiel 12

Zu 33 g des Produktes aus Beispiel 2 und 13,6 g Triethylamin in 200 ml Dichlormethan tropfte man bei 0 °C 8,4 g Oxalsäuredichlorid in 50 ml Dichlormethan. Nach 2 Tagen bei Raumtemperatur wurde abgesaugt, mit Dichlormethan gewaschen, in 250 ml Wasser gelöst, mit Natronlauge alkalisch gestellt und mit n-Butanol extrahiert. Die Butanolphase wurde eingeengt, der ölige Rückstand in 250 ml heißem Methyl-tert.-butylether gelöst und in 1,2 l Petrolether eingetropft. Der ausgefallene Rückstand wurde abgesaugt und in 250 ml Acetonitril gelöst. Bei 5 °C wurde von Unlöslichem filtriert, das Acetonitrilfiltrat eingeengt und der Rückstand aus Essigester umkristallisiert. Man erhielt 6,1 g der Verbindung der Formel 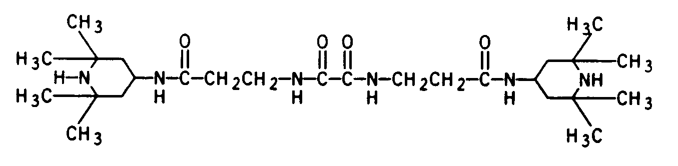
als farblosen Feststoff vom Schmp.: 203°C.

| | | | | | | | | |
|---|---|---|---|---|---|---|---|---|
| Ber.: | C | 61,4 | H | 9,5 | N | 16,5 | O | 12,6 % |
| Gef.: | C | 61,4 | H | 9,7 | N | 16,6 | O | 12,7 % |

### Beispiel 13

Zu 31,0 g des Produktes aus Beispiel 2 und 13,7 g Triethylamin in 100 ml Dichlormethan tropfte man bei 0 °C 11,3 g Adipinsäuredichlorid in 40 ml Dichlormethan. Nach 3 Tagen Rühren bei Raumtemperatur wurde der ausgefallene Niederschlag abgesaugt, mit Dichlormethan gewaschen und in Wasser gelöst. Man stellte mit Natronlauge alkalisch, extrahierte mit n-Butanol und dampfte die organische Phase ein. Nach Umkristallisation des Rückstandes aus Isopropanol unter Zusatz von Aktivkohle erhielt man 11,0 g der Verbindung der Formel 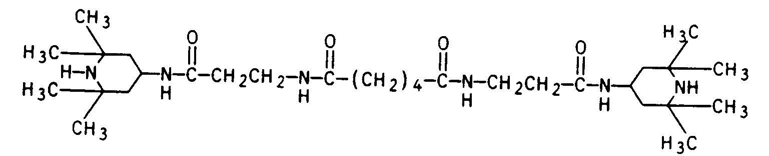
als farblosen Feststoff vom Schmp.: 243°C (Zers.).

| | | | | | | | | |
|---|---|---|---|---|---|---|---|---|
| Ber.: | C | 63,8 | H | 10,0 | N | 14,9 | O | 11,3 % |
| Gef.: | C | 63,4 | H | 9,9 | N | 14,7 | O | 11,4 % |

### Beispiel 14

Zu 22,7 g des Produktes aus Beispiel 2 und 11,0 g Triethylamin in 200 ml Dichlormethan tropfte man 9,9 g 1,5-Pentandicarbonsäuredichlorid in 50 ml Dichlormethan. Nach 3 Stunden Rühren bei Raumtemperatur wurden 500 ml Wasser zugegeben. Nach Phasentrennung wurde die wäßrige Phase mit Natronlauge alkalisch gestellt, mit n-Butanol extrahiert, das n-Butanol abdestilliert. Nach Umkristallisation des Rückstandes aus Toluol erhielt man 3,5 g der Verbindung der Formel 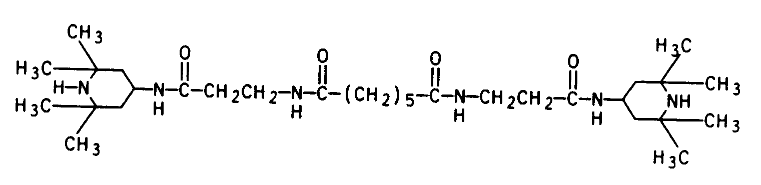
als farblosen Feststoff vom Schmp.: 163 - 165°C

| | | | | | | | | |
|---|---|---|---|---|---|---|---|---|
| Ber.: | C | 64,3 | H | 10,1 | N | 14,5 | O | 11,1 % |
| Gef.: | C | 63,6 | H | 10,0 | N | 14,2 | O | 12,1 % |

Die Verbindung enthielt Kristallwasser.

### Beispiel 15

22,7 g des Produktes aus Beispiel 2 und 11,0 g Triethylamin wurden mit 10,6 g Korksäuredichlorid wie in Beispiel 15 umgesetzt und aufgearbeitet. Nach Umkristallisation aus Toluol erhielt man 12,6 g der Verbindungen der Formel 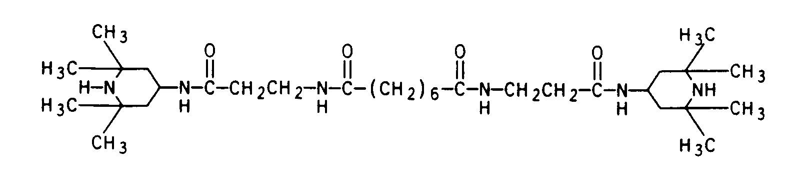
als farblosen Feststoff vom Schmp.: 165 - 169°C.

| | | | | | | | | |
|---|---|---|---|---|---|---|---|---|
| Ber.: | C | 64,8 | H | 10,2 | N | 14,2 | O | 10,8 % |
| Gef.: | C | 63,7 | H | 10,2 | N | 13,7 | O | 12,1 % |

Die Verbindung enthielt Kristallwasser.

### Beispiel 16

Zu 34 g des Produktes aus Beispiel 2 in 125 ml Dichlormethan tropfte man bei 0 °C 17,0 g Sebacinsäuredichlorid in 50 ml Dichlormethan und 15,1 g Triethylamin in 40 ml Dichlormethan. Nach 2 Tagen Rühren bei Raumtemperatur wurde abgesaugt, der Rückstand in 250 ml Wasser gelöst. Nachdem die wäßrige Phase mit Natronlauge alkalisch gestellt wurde, wurde mit n-Butanol extrahiert. Nach Abdampfen des n-Butanols blieben als Rückstand 39 g der Verbindung der Formel 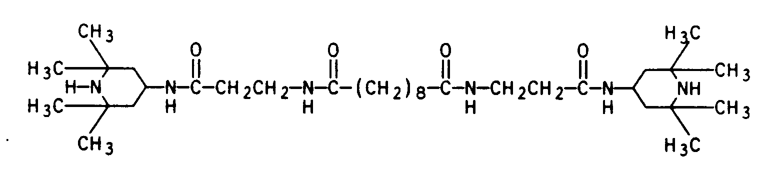
als farbloser Feststoff vom Schmp.: 144°C.

| | | | | | | | | |
|---|---|---|---|---|---|---|---|---|
| Ber.: | C | 65,8 | H | 10,4 | N | 13,5 | O | 10,3 % |
| Gef.: | C | 65,1 | H | 10,5 | N | 13,0 | O | 11,2 % |

Die Verbindung enthielt Kristallwasser.

### Beispiel 17

32,7 g des Produktes aus Beispiel 2 und 13,5 g Triethylamin wurden mit 13,2 g Terephthalsäuredichlorid wie in Beispiel 16 umgesetzt und aufgearbeitet. Umkristallisation aus Isopropanol-n-Butanol (1:1) lieferte 19 g der Verbindung der Formel 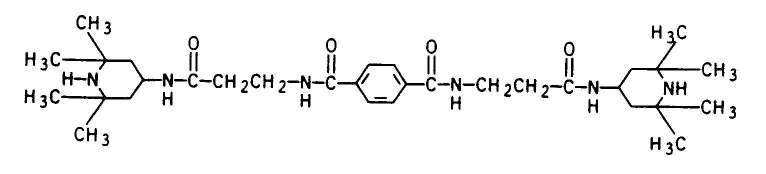
als farblosen Feststoff vom Schmp.: 262 - 263°C.

| | | | | | | | | |
|---|---|---|---|---|---|---|---|---|
| Ber.: | C | 65,7 | H | 9,0 | N | 14,4 | O | 10,9 % |
| Gef.: | C | 65,2 | H | 9,1 | N | 14,2 | O | 11,3 % |

### Beispiel 18

Zu 41 g des Produktes aus Beispiel 2 und 15 g Triethylamin in 250 ml Dichlormethan tropfte man bei 0 °C 18,1 g n-Propylchlorformiat in 50 ml Dichlormethan. Nach 16 Stunden Rühren bei Raumtemperatur wurde das Lösungsmittel abgedampft, der Rückstand in Wasser gelöst. Die wäßrige Phase wurde mit Natronlauge alkalisch gestellt und mit n-Butanol extrahiert. Nach Abdestillieren des n-Butanols wurde der Rückstand zweimal aus Methyl-tert.-butylether und anschließend aus Aceton umkristallisiert. Man erhielt 18,0 g der Verbindung der Formel 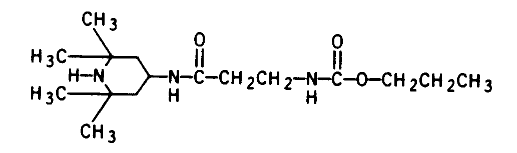
als farblosen Feststoff vom Schmp.: 116°C.

| | | | | | | | | |
|---|---|---|---|---|---|---|---|---|
| Ber.: | C | 61,3 | H | 10,0 | N | 13,4 | O | 15,3 % |
| Gef.: | C | 61,3 | H | 10,0 | N | 13,5 | O | 15,1 % |

### Beispiel 19

41 g des Produktes aus Beispiel 2 und 17,8 g Triethylamin wurden mit 24 g n-Butylchlorformiat wie in Beispiel 18 umgesetzt und aufgearbeitet. Nach Umkristallisation aus Acetonitril und Waschen mit Petrolether erhielt man 22 g der Verbindung der Formel 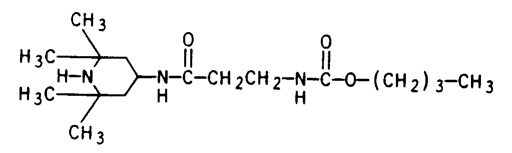
als farblosen Feststoff vom Schmp.: 76°C.

| | | | | | | | | |
|---|---|---|---|---|---|---|---|---|
| Ber.: | C | 62,3 | H | 10,1 | N | 12,8 | O | 14,6 % |
| Gef.: | C | 62,2 | H | 10,2 | N | 12,9 | O | 15,2 % |

### Beispiel 20

Zu 34 g des Produktes aus Beispiel 2 und 15,2 g Triethylamin in 200 ml Dichlormethan tropfte man bei 0 °C 41,5 g Myristylchlorformiat in 100 ml Dichlormethan. Nach 16 Stunden Rühren bei Raumtemperatur wurden 100 ml Wasser zugegeben, mit Natronlauge alkalisch gestellt und die Phasen getrennt. Die wäßrige Phase wurde mit n-Butanol extrahiert, die organischen Phasen wurden vereinigt und eingedampft. Der Rückstand lieferte nach Umkristallisation aus n-Heptan 56 g der Verbindung der Formel 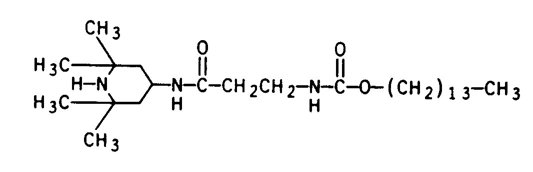
als farblosen Feststoff vom Schmp.: 58°C.

| | | | | | | | | |
|---|---|---|---|---|---|---|---|---|
| Ber.: | C | 69,3 | H | 11,4 | N | 8,9 | O | 10,3 % |
| Gef.: | C | 68,9 | H | 11,4 | N | 8,8 | O | 10,4 % |

### Beispiel 21

34 g des Produktes aus Beispiel 2 und 15,2 g Triethylamin wurden mit 45,7 g Cetylchlorformiat wie in Beispiel 20 umgesetzt und aufgearbeitet. Umkristallisation aus n-Heptan lieferte 61 g der Verbindung der Formel 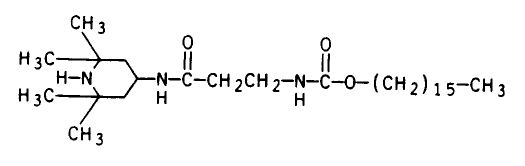
als farblosen Feststoff von Schmp.: 64°C.

| | | | | | | | | |
|---|---|---|---|---|---|---|---|---|
| Ber.: | C | 70,2 | H | 11,6 | N | 8,5 | O | 9,7 % |
| Gef.: | C | 70,0 | H | 11,5 | N | 8,4 | O | 9,9 % |

### Beispiel 22

34 g des Produktes aus Beispiel 2 und 15,2 g Triethylamin wurden mit 28,9 g 2-Ethylhexylchlorformiat wie in Beispiel 20 umgesetzt und aufgearbeitet. Das nach dem Abdampfen des Lösungsmittels erhaltene Öl war nicht destillierbar. Es wurde in Dichlormethan gelöst und 16 Stunden mit Aktivkohle gerührt. Nach dem Abdampfen des Lösungsmittels erhielt man 42 g der Verbindung der Formel 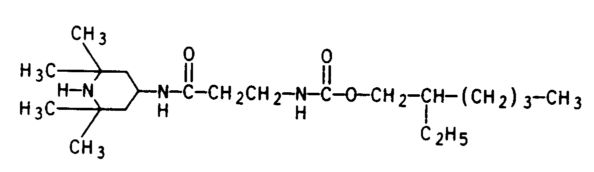
als viskoses, leicht gelbliches Öl.

| | | | | | | | | |
|---|---|---|---|---|---|---|---|---|
| Ber.: | C | 65,8 | H | 10,8 | N | 10,9 | O | 12,5 % |
| Gef.: | C | 65,4 | H | 10.7 | N | 11,2 | O | 12,7 % |

### Beispiel 23

34,5 g des Produktes aus Beispiel 2 und 15,1 g Triethylamin wurden mit 20,8 g 2-Methoxyethylchlorformiat wie in Beispiel 22 umgesetzt und aufgearbeitet. Man erhielt 31 g der Verbindung der Formel 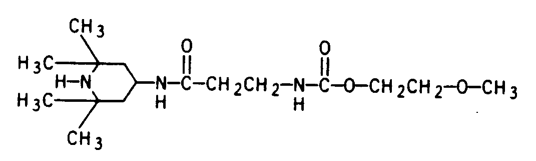
als hochviskoses, nicht destillierbares, leicht gelbliches Öl.

| | | | | | | | | |
|---|---|---|---|---|---|---|---|---|
| Ber.: | C | 58,3 | H | 9,5 | N | 12,7 | O | 19,4 % |
| Gef.: | C | 58,3 | H | 9,5 | N | 12,7 | O | 19,5 % |

### Beispiel 24

Zu 34,0 g des Produkts aus Beispiel 2 und 15 g Triethylamin in 200 ml Dichlormethan tropfte man das aus 19,5 g Heptansäure und 23,6 g Thionylchlorid hergestellte Säurechlorid in 100 ml Dichlormethan. Nach 4 Stunden Rühren bei Raumtemperatur gab man Eiswasser zu, stellte mit Natronlauge alkalisch, trennte die organische Phase ab und trocknete über Magnesiumsulfat. Nach dem Abdampfen des Lösungsmittels wurde der Rückstand aus Acetonitril umkristallisiert. Man erhielt 30,4 g der Verbindung der Formel 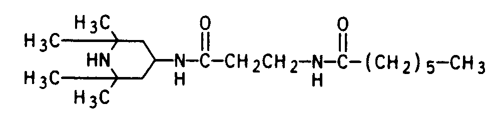
als farblosen Festoff vom Schmp.: 112°C.

| | | | | | | | | |
|---|---|---|---|---|---|---|---|---|
| Ber.: | C | 67,2 | H | 11,0 | N | 12,4 | O | 9,4 % |
| Gef.: | C | 67,1 | H | 11,0 | N | 12,3 | O | 9,3 % |

### Beispiel 25

34 g des Produktes aus Beispiel 2, 15 g Triethylamin und das aus 30 g Laurinsäure und 23,6 g Thionylchlorid hergestellte Säurechlorid wurden wie in Beispiel 24 umgesetzt und aufgearbeitet. Man erhielt 45 g der Verbindung der Formel 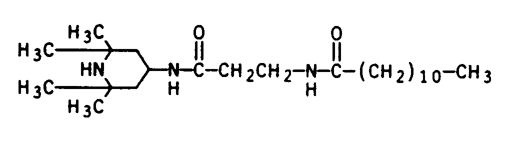
als farblosen Feststoff vom Schmp.: 68°C.

| | | | | | | | | |
|---|---|---|---|---|---|---|---|---|
| Ber.: | C | 70,4 | H | 11,6 | N | 10,3 | O | 7,8 % |
| Gef.: | C | 69,8 | H | 11,5 | N | 10,6 | O | 8,0 % |

### Beispiel 26

34 g des Produktes aus Beispiel 2, 15 g Triethylamin und das aus 42,7 g Stearinsäure und 23,6 g Thionylchlorid wurden wie in Beispiel 24 umgesetzt und aufgearbeitet. Man erhielt 51,3 g der Verbindung der Formel 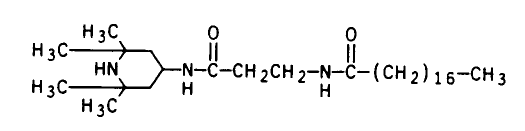
als farblosen Feststoff vom Schmp.: 80°C.

| | | | | | | | | |
|---|---|---|---|---|---|---|---|---|
| Ber.: | C | 73,0 | H | 12,0 | N | 8,5 | O | 6,5 % |
| Gef.: | C | 72,4 | H | 12,0 | N | 8,4 | O | 6,7 % |

### Beispiel 27

34 g des Produktes aus Beispiel 2, 15 g Triethylamin und das aus 20,4 g 2-Methylbenzoesäure und 23,6 g Thionylchlorid hergestellte Säurechlorid wurden wie in Beispiel 24 umgesetzt und aufgearbeitet. Man erhielt 31,1 g der Verbindung der Formel 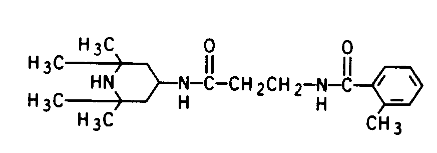
als farblosen Feststoff vom Schmp.: 191°C.

| | | | | | | | | |
|---|---|---|---|---|---|---|---|---|
| Ber.: | C | 69,5 | H | 9,0 | N | 12,2 | O | 9,3 % |
| Gef.: | C | 69,2 | H | 9,1 | N | 12,2 | O | 9,4 % |

### Beispiel 28

Zu 34 g des Produktes aus Beispiel 2 und 15 g Triethylamin in 400 ml Dichlormethan tropfte man bei 0 °C 26,3 g 4-Chlorbenzoylchlorid in 100 ml Dichlormethan. Nach 4 Stunden Rühren bei Raumtemperatur wurde der ausgefallene Niederschlag abgesaugt, mit wenig Dichlormethan gewaschen und getrocknet. Man löste ihn in Wasser, stellte mit Natronlauge basisch und extrahierte mit Dichlormethan. Nach dem Abdampfen des Lösungsmittels wurde der Rückstand aus Acetonitril umkristallisiert. Man erhielt 24,1 g der Verbindung der Formel 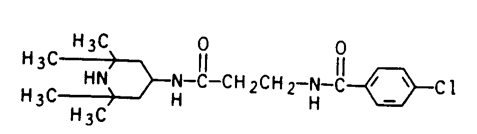
als farblosen Feststoff vom Schmp. 172°C.

| | | | | | | | | | | |
|---|---|---|---|---|---|---|---|---|---|---|
| Ber.: | C | 62,4 | H | 7,7 | Cl | 9,7 | N | 11,5 | O | 8,7 % |
| Gef.: | C | 62,4 | H | 7,8 | Cl | 9,3 | N | 11,6 | O | 8,8 % |

### Beispiel 29

12,5 g des Produktes aus Beispiel 2 und 7,4 g Phthalsäureanhydrid wurden in 150 ml Toluol 7 Stunden am Wasserauskreiser gekocht. Der ausgefallene Niederschlag wurde heiß abgesaugt und getrocknet. Er bestand aus 17,3 g der Verbindung der Formel 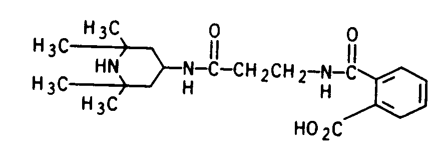
vom Schmp.: 212 - 214°C (Zers.).

| | | | | | | | | |
|---|---|---|---|---|---|---|---|---|
| Ber.: | C | 64,0 | H | 7,8 | N | 11.2 | O | 17,0 % |
| Gef.: | C | 63,9 | H | 8,1 | N | 11,2 | O | 16,7 % |

### Beispiel 30

34 g des Produktes aus Beispiel 2, 15 g Triethylamin und das aus 20,4 g 4-Methylbenzoesäure und 23,6 g Thionylchlorid hergestellte Säurechlorid wurden wie in Beispiel 24 umgesetzt. Nach 4 Stunden wurde filtriert, das Filtrat mit Eiswasser und Natronlauge versetzt und mit Dichlormethan extrahiert. Nach dem Abdestillieren des Dichlormethans wurde der Rückstand aus Acetonitril umkristallisiert. Man erhielt 13,8 der Verbindung der Formel 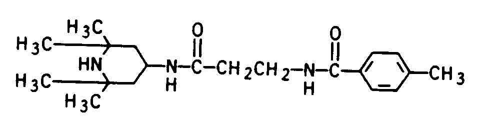
als farblosen Feststoff vom Schmp.: 159 - 160°C.

| | | | | | | | | |
|---|---|---|---|---|---|---|---|---|
| Ber.: | C | 69,5 | H | 9,1 | N | 12,2 | O | 9,3 % |
| Gef.: | C | 69,4 | H | 9,3 | N | 12,3 | O | 8,8 % |

### Beispiel 31

34 g des Produktes aus Beispiel 2, 15 g Triethylamin und das aus 21,6 g Octansäure und 23,6 g Thionylchlorid hergestellt Säurechlorid wurden wie in Beispiel 24 umgesetzt und aufgearbeitet. Nach Umkristallistion aus Acetonitril erhielt man 20,7 g der Verbindung der Formel 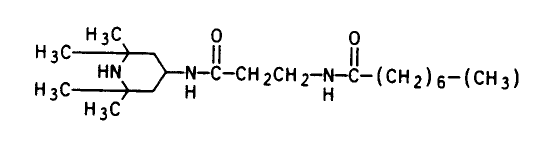
als farblosen Feststoff vom Schmp.: 84°C.

| | | | | | | | | |
|---|---|---|---|---|---|---|---|---|
| Ber.: | C | 67,9 | H | 11,1 | N | 11,9 | O | 9,1 % |
| Gef.: | C | 67,8 | H | 11,2 | N | 12,0 | O | 9,1 % |

### Beispiel 32

45,4 g des Produktes aus Beispiel 2 und 42,9 g Undecansäureethylester wurden in 200 ml Ethanol 2 Stunden gekocht. Nach Zugabe von 35 ml 30 %iger methanolischer Natriummethylatlösung wurde weitere 8 Stunden zum Sieden erhitzt. Nach dem Einengen wurde der Rückstand mit 300 ml Acetonitril min. gerührt. Man filtrierte von Unlöslichem ab, das Filtrat wurde eingeengt und der dabei erhaltene Rückstand in 500 ml Wasser und 250 ml Dichlormethan verteilt. Nach der Phasentrennung wurde die Dichlormethanphase vom Lösungsmittel befreit. Man erhielt als Rückstand 32,8 g der Verbindung der Formel 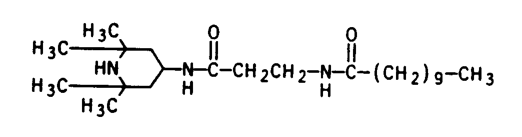
als zähflüssige Masse, die nach wenigen Tagen zu einem farblosen Feststoff vom Schmp.: 72 - 75°C erstarrt.

| | | | | | | | | |
|---|---|---|---|---|---|---|---|---|
| Ber.: | C | 69,8 | H | 11,5 | N | 10,6 | O | 8,3 % |
| Gef.: | C | 69,4 | H | 11,5 | N | 10,9 | O | 8,3 % |

### Beispiel 33

34 g des Produktes aus Beispiel 2, 15 g Triethylamin und das aus 22,8 g 4-Methoxybenzoesäure hergestellte Säurechlorid wurden wie in Beispiel 24 umgesetzt und aufgearbeitet. Nach Umkristallistion aus Acetonitril erhielt man 30 g der Verbindung der Formel 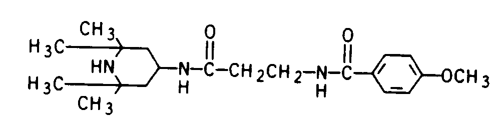
als farblosen Feststoff vom Schmp.: 145 bis 147°C.

| | | | | | | | | |
|---|---|---|---|---|---|---|---|---|
| Ber.: | C | 66,5 | H | 8,6 | N | 11,6 | O | 13,3 % |
| Gef.: | C | 66,3 | H | 8,6 | N | 11,4 | O | 13,2 % |

### Beispiel 34

34 g des Produktes aus Beispiel 2, 15 g Triethylamin und das aus 21 g 2,4-Dimethyl-furan-3-carbonsäure und 23,6 g Thionylchlorid hergestellte Säurechlorid wurden wie in Beispiel 28 umgesetzt und aufgearbeitet. Nach Umkristallistion aus Acetonitril erhielt man 15,2 g der Verbindung der Formel 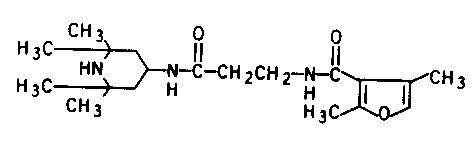
als farblosen Feststoff vom Schmp.: 176°C.

| | | | | | | | | |
|---|---|---|---|---|---|---|---|---|
| Ber.: | C | 65,3 | H | 8,9 | N | 12,0 | O | 13,8 % |
| Gef.: | C | 64,7 | H | 9,0 | N | 11,9 | O | 13,9 % |

### Beispiel 35

46,3 g des Produktes aus Beispiel 2 wurden in 250 ml Ameisensäuremethylester unter Zusatz von 3,6 g 30 %iger methanolischer Natriummethylatlösung 9 h gekocht. Der ausgefallene Niederschlag wurde abgesaugt, in Wasser gelöst und die Wasserphase mit n-Butanol extrahiert. Nach dem Einengen der n-Butanolphase wurde der Rückstand aus Acetonitril/iso-Propanol (6:1) umkristallisiert. Man erhielt 19 g der Verbindung der Formel 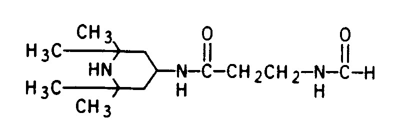
als farblosen Feststoff vom Schmp.: 211°C.

| | | | | | | | | |
|---|---|---|---|---|---|---|---|---|
| Ber.: | C | 61,6 | H | 9,9 | N | 16,4 | O | 12,5 % |
| Gef.: | C | 60,8 | H | 10,0 | N | 16,6 | O | 13,0 % |

### Beispiel 36

34 g des Produktes aus Beispiel 2, 15 g Triethylamin und das aus 17, 1 g Cyclopentancarbonsäure und 23,6 g Thionylchlorid hergestellte Säurechlorid wurden wie in Beispiel 24 umgesetzt und aufgearbeitet. Nach Umkristallisation aus Acetonitril erhielt man 14 g der Verbindung der Formel 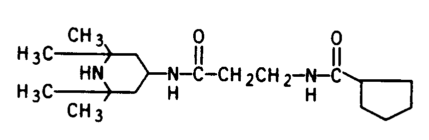
als farblosen Feststoff vom Schmp.: 190°C.

| | | | | | | | | |
|---|---|---|---|---|---|---|---|---|
| Ber.: | C | 66,8 | H | 10,3 | N | 13,0 | O | 9,9 % |
| Gef.: | C | 66,4 | H | 10,3 | N | 12,8 | O | 10,0 % |

### Beispiel 37

34 g des Produktes aus Beispiel 2, 15 g Triethylamin und 23,8 g 2,5-Dimethylfuran-3-carbonsäurechlorid wurden wie in Beispiel 24 umgesetzt und aufgearbeitet. Nach Umkristallisation aus Acetonitril erhielt man 25,9 g der Verbindung der Formel 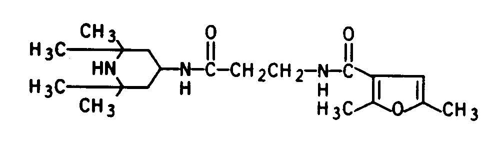
als farblosen Feststoff vom Schmp.: 193 bis 194°C.

| | | | | |
|---|---|---|---|---|
| Ber.: | C 65,3 | H 8,9 | N 12,0 | O 13,8 % |
| Gef.: | C 65,3 | 8,98,6 | N 11,9 | O 13,8 % |

### Beispiel 38

34 g des Produktes aus Beispiel 2, 15 g Triethylamin und das aus 20,4 g Phenylessigsäure und 23,6 g Thionylchlorid hergestellte Säurechlorid wurden wie in Beispiel 24 umgesetzt und aufgearbeitet. Nach Umkristallisation aus Acetonitril erhielt man 12,7 g der Verbindung der Formel 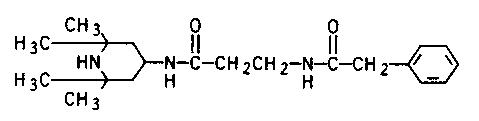
als farblosen Feststoff vom Schmp.: 187 bis 188°C.

| | | | | | | | | |
|---|---|---|---|---|---|---|---|---|
| Ber.: | C | 69,5 | H | 9,0 | N | 12,2 | O | 9,3 % |
| Gef.: | C | 67,3 | H | 8,9 | N | 11,7 | O | 12,0 % |

### Beispiel 39

34 g des Produktes aus Beispiel 2, 15 g Triethylamin und das aus 32,2 g Tridecansäure und 23,6 g Thionylchlorid hergestellte Säurechlorid wurden wie in Beispiel 24 umgesetzt und aufgearbeitet. Nach Umkristallisation aus Acetonitril erhielt man 38 g der Verbindung der Formel 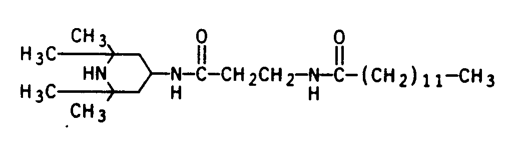
als farblosen Feststoff vom Schmp.: 82°C.

| | | | | | | | | |
|---|---|---|---|---|---|---|---|---|
| Ber.: | C | 70,8 | H | 11,6 | N | 10,0 | O | 7,6 % |
| Gef.: | C | 69,9 | H | 11,4 | N | 9,9 | O | 7,6 % |

### Beispiel 40

34 g des Produktes aus Beispiel 2, 15 g Triethylamin und das aus 22,8 g Phenoxyessigsäure und 23,6 g Thionylchlorid hergestellte Säurechlorid wurden wie in Beispiel 24 umgesetzt und aufgearbeitet. Nach Umkristallisation aus Acetonitril erhielt man 18,4 g der Verbindung der Formel 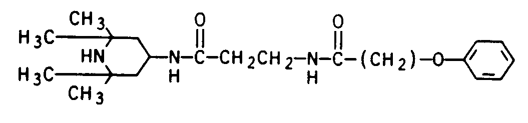
als farblosen Feststoff vom Schmp.: 128 bis 130°C.

| | | | | | | | | |
|---|---|---|---|---|---|---|---|---|
| Ber.: | C | 66,5 | H | 8,6 | N | 11,6 | O | 13,3 % |
| Gef.: | C | 65,6 | H | 8,4 | N | 11,4 | O | 13,8 % |

### Beispiel 41

34,5 g des Produktes aus Beispiel 2, 15,2 g Triethylamin und 25,6 g Benzylchlorformiat wurden wie in Beispiel 20 umgesetzt und aufgearbeitet. Der Rückstand, der nach dem Einengen der organischen Phase erhalten wurde, wurde in heißem Essigester gelöst und mit Aktivkohle behandelt. Nach dem Filtrieren und Einengen der Essigesterphase erhielt man 36 g der Verbindung der Formel 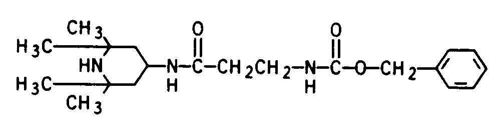
als leicht klebriges Harz.

| | | | | | | | | |
|---|---|---|---|---|---|---|---|---|
| Ber.: | C | 66,4 | H | 8,6 | N | 11,6 | O | 13,3 % |
| Gef.: | C | 66,3 | H | 8,7 | N | 11,5 | O | 14,2 % |

### Beispiel 42

34 g des Produktes aus Beispiel 2, 15,1 g Triethylamin und das aus 20,5 g Isobutylchlorformiat wurden wie in Beispiel 41 umgesetzt und aufgearbeitet. Man erhielt 32 g der Verbindung der Formel 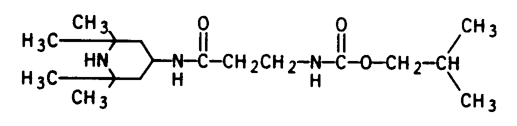
als leicht klebriges Harz.

| | | | | | | | | |
|---|---|---|---|---|---|---|---|---|
| Ber.: | C | 62,3 | H | 10,1 | N | 12,8 | O | 14,6 % |
| Gef.: | C | 62,4 | H | 10,3 | N | 12,6 | O | 15,1 % |

### Beispiel 43

34,5 g des Produktes aus Beispiel 2, 15 g Triethylamin und 24,7 g Hexylchlorformiat wurden wie in Beispiel 41 umgesetzt und aufgearbeitet. Man erhielt 41 g der Verbindung der Formel 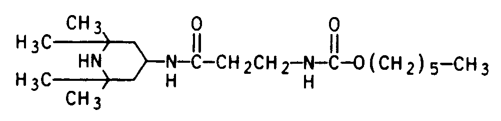
als leicht klebriges Harz.

| | | | | | | | | |
|---|---|---|---|---|---|---|---|---|
| Ber.: | C | 64,2 | H | 10,5 | N | 11,8 | O | 13,5 % |
| Gef.: | C | 63,9 | H | 10,4 | N | 11,9 | O | 14,1 % |

### Beispiel 44

34,5 g des Produktes aus Beispiel 2, 15,2 g Triethylamin und 15,2 g Triethylamin und 24,4 g Cyclohexylchlorformiat wurden wie in Beispiel 20 umgesetzt und aufgearbeitet. Ölpumpenvakuumdestillation lieferte 25,7 g der Verbindung der Formel 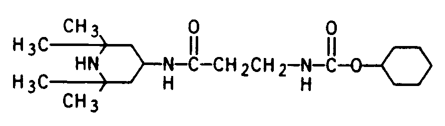
als farbloses Öl vom Schmp. 222 bis 224°C/0,3 Torr, das zu einem farblosen Feststoff erstarrte und nach Umkristallisation aus n-Heptan bei 110°C schmolz.

| | | | | | | | | |
|---|---|---|---|---|---|---|---|---|
| Ber.: | C | 64,5 | H | 10,0 | N | 11,9 | O | 13,6 % |
| Gef.: | C | 64,2 | H | 10,0 | N | 12,3 | O | 13,3 % |

### Beispiel 45

34,2 g des Produktes aus Beispiel 2, 15 g Triethylamin und 18,2 g 1,6-Hexandiol-bis-chlorformiat wurden wie in Beispiel 41 umgesetzt und aufgearbeitet. Man erhielt 26 g der Verbindung der Formel 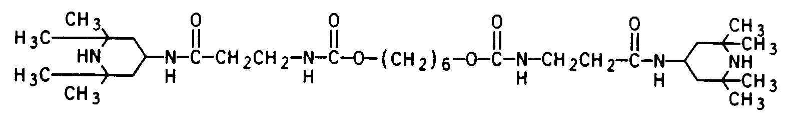
als farblosen Feststoff vom Schmp.: 62°C.

| | | | | | | | | |
|---|---|---|---|---|---|---|---|---|
| Ber.: | C | 61,5 | H | 9,7 | N | 13,4 | O | 15,4 % |
| Gef.: | C | 61,1 | H | 9,9 | N | 12,7 | O | 16,1 % |

### Beispiel 46

37 g Phtalsäureanhydrid, 56,8 g des Produktes aus Beispiel 2 und 16 g Lewatit® S 100 wurden in 300 ml Xyxlol am Wasserabscheider gekocht, bis sich kein Wasser mehr abschied. Man filtrierte heiß und ließ auf Raumtemperatur abkühlen. Der ausgefallene Niederschlag bestand aus 36,8 g der Verbindung der Formel

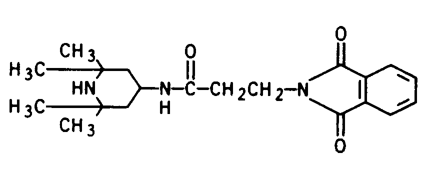

Die farblosen Kristalle schmolzen bei 188°C.

| | | | | | | | | |
|---|---|---|---|---|---|---|---|---|
| Ber.: | C | 67,2 | H | 7,6 | N | 11,8 | O | 13,4 % |
| Gef.: | C | 65,9 | H | 7,7 | N | 11,4 | O | 14,4 % |

Die Verbindung enthält 0,5 Mol Kristallwasser.

### Beispiel 47

34 g des Produktes aus Beispiel 2, 15 g Triethylamin und 29,5 g 4-tertiär-Buty-benzoesäurechlorid wurden wie in Beispiel 16 umgesetzt und aufgearbeitet. Umkristallisation aus Toluol lieferte 31,4 g der Verbindung der Formel 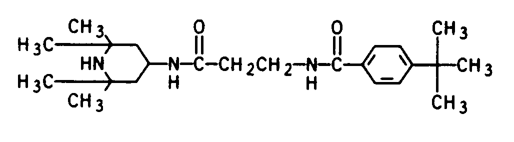
als farblosen Feststoff vom Schmp. 105 bis 107°C.

### Beispiel 48

34 g des Produktes aus Beispiel 2, 15 g Triethylamin und das aus 19,3 g Cyclohexancarbonsäure und 23,6 g Thionylchlorid hergestellte Säurechlorid wurden wie in Beispiel 16 umgesetzt und aufgearbeitet. Umkristallisation aus Essigsäureethylester lieferte 16,4 g der Verbindung der Formel 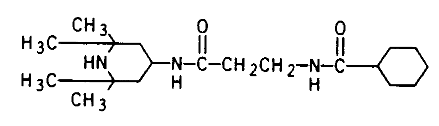
als farblosen Feststoff vom Schmp. 175 bis 178°C.

### Beispiel 49

34 g des Produktes aus Beispiel 2, 15 g Triethylamin und das aus 24,9 g 4-Methoxyphenylessigsäure und 23,6 g Thionylchlorid hergestellte Säurechlorid wurden wie in Beispiel 24 umgesetzt und aufgearbeitet. Umkristallisation aus Acetonitril lieferte 24 g der Verbindung der Formel 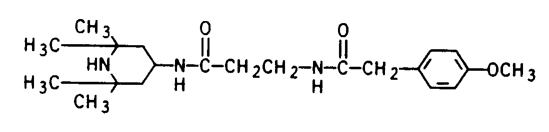
als farblosen Feststoff vom Schmp. 168 bis 169°C.

### Beispiel 50

68 g des Produktes aus Beispiel 2, 30 g Triethylamin und das aus 36,6 g 1,11-Undecandicarbonsäure und 47,2 g Thionylchlorid hergestellte Säurechlorid wurden wie in Beispiel 24 umgesetzt und aufgearbeitet. Umkristallisation aus Acetonitril lieferte 52,8 g der Verbindung der Formel 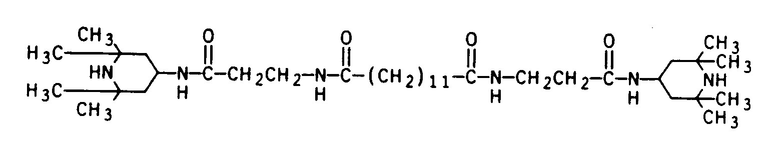
als farblosen Feststoff vom Schmp. 65 bis 67°C.

### Beispiel 51

Zu einer Lösung von 34 g des Produktes aus Beispiel 2 und 15 g Triethylamin in 400 ml Dichlormethan wurden 16,9 g Nonandisäurechlorid gegeben. Nach 16 h Rühren wurde auf Eiswasser gegeben, mit Natronlauge alkalisch gestellt und nach der Phasentrennung die organische Phase eingeengt. Der verbleibende Rückstand wurde aus Acetonitril umkristallisiert. Man erhielt 5,6 g der Verbindung der Formel 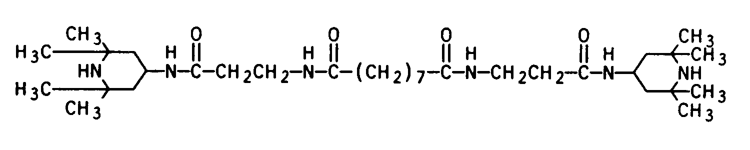
als farbloses Dihydrat vom Schmp. 90 bis 94°C.

| | | | | | | | | |
|---|---|---|---|---|---|---|---|---|
| Ber.: | C | 61,6 | H | 10,3 | N | 13,1 | O | 14,9 |
| Gef.: | C | 61,6 | H | 10,4 | N | 12,6 | O | 14,8 |

### Beispiel 52

Zu einer Lösung von 34 g des Produktes aus Beispiel 2 und 15 g Triethylamin in 200 ml Dichlormethan wurde eine Lösung von 15,7 g Cyclopropancarbonsäurechlorid in 100 ml Dichlormethan getropft. Nach 16 h Rühren wurde mit Eiswasser versetzt, mit Natronlauge alkalisch gestellt, die organische Phase abgetrennt und die wäßrige Phase mit n-Butanol ausgeschüttelt. Die organischen Phasen wurden eingeengt, man erhielt 36 g Rohprodukt vom Schmp. 175 bis 178°C. Nach Umkristallisation aus Essigsäureethylester erhielt man 12,8 g der Verbindung der Formel 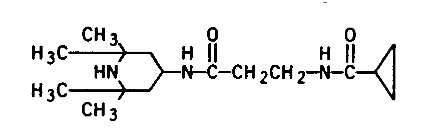
vom Schmp. 187 bis 188°C.

| | | | | | | | | |
|---|---|---|---|---|---|---|---|---|
| Ber.: | C | 65,1 | H | 9,9 | N | 14,2 | O | 10,8 |
| Gef.: | C | 64,8 | H | 10,0 | N | 14,1 | O | 11,0 |

### Beispiel 53

56,8 g des Produktes aus Beispiel 1 und 49,5 g 1,8-Naphthalindicarbonsäureanhydrid wurden in 300 ml Xylol 36 h am Wasserabscheider gekocht. Nach Filtrieren in der Hitze wurde der ausgefallene Niederschlag abgesaugt; man erhielt 70 g Rohprodukt vom Schmp. 178 bis 180°C. Nach Umkristallisation aus Acetonitril erhielt man 32,6 g der Verbindung der Formel 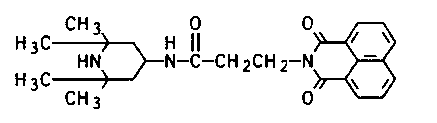
als farbloses Halbhydrat vom Schmp. 182°C.

| | | | | | | | | |
|---|---|---|---|---|---|---|---|---|
| Ber.: | C | 69,2 | H | 7,3 | N | 10,1 | O | 13,4 |
| Gef.: | C | 68,9 | H | 7,3 | N | 10,4 | O | 13,2 |

### Anwendungsbeispiel

### Beispiel 1

### Stabilisierung von Polyamid

a) 0,2 Teile des Produktes aus Beispiel 16 wurde in 100 Teilen marktübliches Polyamid (Ultramid B3S der Firma BASF) durch einmaliges Extrudieren bei 250 °C eingearbeitet und das anfallende Granulat über eine Spritzgießmaschine bei 250 °C zu 2 mm dicken Prüfkörpern gespritzt.
b) Die nach a) hergestellen Prüflinge wurden in einem Xenotest® 1200 - Schnellbewitterungsgerät auf ihre Licht- und Wetterechtheit getestet. Die Alterung wurde durch Messen der Zeit bis zum Beginn der Rißbildung an der Oberfläche des Prüfkörpers bestimmt.

Die nach a) hergestellten Prüflinge wiesen nach 2000 Stunden Bewitterung noch keine Risse in der Oberfläche auf, während analoge Prüflinge ohne Stabilisator schon nach 750 Stunden Bewitterung Risse aufwiesen.

## Patentansprüche

1. Verbindungen der allgemeinen Formel (I) 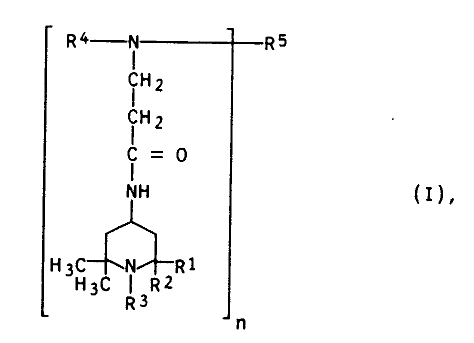 in der
R¹ und R² Methyl,
R³ Wasserstoff, C₁- bis C₈-Alkyl, C₁- bis C₈-Alkoxycarbonyl, C₁-C₈-Alkanoyl, Benzyl, Hydroxyethyl, Cyanmethyl oder Aminoethyl und
n 1, 2 oder 3 bedeuten, und -
wenn n = 1 ist - stehen
R⁴ fürWasserstoff und
R⁵
a) für 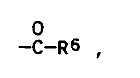 worin
R⁶ Wasserstoff, C₁-C₁₉-Alkyl, C₇-C₁₀-Phenylalkyl, worin der Phenylkern gegebenenfalls durch 1, 2 oder 3 C₁-C₈-Alkyl, Chlor, Brom, Fluor, Hydroxy, C₁-C₄-Alkoxy, Phenyl, Phenoxy oder Carboxy substituiert ist und die Substituenten gleich oder verschieden sein können, Phenoxy-C₁-C₄-alkyl, gegebenenfalls durch 1 bis 3 C₁-C₈-Alkyl substituiertes C₃-C₁₀-Cycloalkyl; gegebenenfalls durch C₁-C₈-Alkyl, Chlor, Brom, Fluor, Hydroxy, C₁-C₄-Alkoxy, Phenyl, Phenoxy oder Carboxyl substituiertes Phenyl oder Naphthyl und worin die Zahl der Substituenten bis zu 3 betragen kann; einen 5- oder 6-gliedrigen gesättigten oder aromatischen O, S oder N enthaltenden Heterocyclus, der gegebenenfalls durch 1 bis 4 C₁-C₄-Alkyl substituiert ist; C₁-C₁₈-Alkoxy, worin die Alkylkette durch -O- unterbrochen sein kann; Phenoxy, Naphthoxy, gegebenenfalls durch 1 oder 2 C₁-C₄-Alkyl substituiertes C₅- oder C₆-Cycloalkoxy oder Phenylalkoxy mit insgesamt 7 bis 12C-Atomen ist,
b) für -CO-NH-R⁷, worin R⁷ Wasserstoff, C₁-C₁₈-Alkyl, C₅- oder C₆-Cycloaklyl, Phenyl, das gegebenenfalls durch 1 bis 3 C₁-C₈-Alkyl, Chlor, Brom, Fluor oder C₁-C₄-Alkoxy substituiert ist, oder Phenyl-C₁-C₄-alkyl ist oder
c) für -SO₂- R⁸, worin R⁸ C₁-C₁₈-Alkyl oder gegebenenfalls durch C₁-C₄-Alkyl oder Chlor substituiertes Phenyl ist, oder 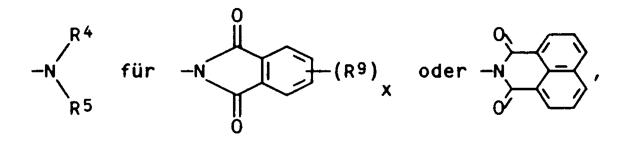 worin R⁹ C₁-C₈-Alkyl, Chlor, Brom oder Fluor und x = 0, 1, 2, 3 oder 4 bedeuten, oder - wenn n = 2 ist - stehen
R⁴ für Wasserstoff und
R⁵ für 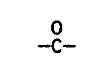 oder 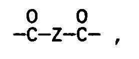 worin
Z eine chemische Bindung, C₁-C₁₂-Alkylen, C₄-C₈-Oxaalkylen, Phenylen, Cyclohexylen, Diphenylen, Diphenylenoxid, Dioxyphenylen, Diaminophenylen, Diaminocyclohexylen, 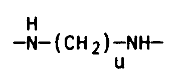 mit u = 2 bis 6, -O-X-O-, worin X C₂-C₈-Alkylen oder C₄-C₈-Oxaalkylen oder ein zweiwertiger gesättigter oder ungesättigter 5- oder 6-gliedriger O, S oder N enthaltender Heterocyclus ist, oder 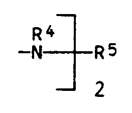 für eine Pyromellithsäurediimidgruppe,
oder - wenn n = 3 ist - stehen
R⁴ für Wasserstoff und
R⁵ für 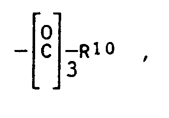 worin
R¹⁰ einen dreiwertigen C₁-C₈-Alkylrest oder einen dreiwertigen Phenylrest bedeutet,
sowie Säureadditionssalze und Hydrate der Verbindungen (I).

2. Verbindungen nach Anspruch 1, dadurch gekennzeichnet, daß R³ Wasserstoff bedeutet.

3. Verwendung von Verbindungen gemäß Anspruch 1 oder 2 zum Stabilisieren von organischem Material, insbesondere von Kunststoffen.

4. Verwendung von Verbindungen gemäß Anspruch 1 oder 2 zum Stabilisieren von Polyurethanen.

5. Verwendung von Verbindungen gemäß Anspruch 1 oder 2 zum Stabilisieren von Polyolefinen.

6. Verwendung von Verbindungen gemäß Anspruch 1 oder 2 zum Stabilisieren von Polyamid.

7. Verwendung von Verbindungen gemäß Anspruch 1 oder 2 zum Stabilisieren von Lacken.

8. Verwendung von Verbindungen gemäß Anspruch 1 oder 2 als Metallionendesaktivator.

9. Stabilisiertes organisches Material, enthaltend Verbindungen gemäß Anspruch 1 oder 2.

## Claims

1. A compound of the general formula (I) 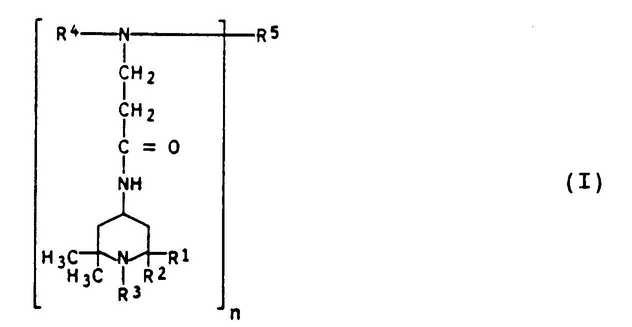 where
R¹ and R² are each methyl,
R³ is hydrogen, C₁-C₈-alkyl, C₁-C₈-alkoxycarbonyl, C₁-C₈-alkanoyl, benzyl, hydroxyethyl, cyanomethyl or aminoethyl,
n is 1, 2 or 3, and -
when n is 1 -
R⁴ is hydrogen and
R⁵ is
a) 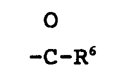 where
R⁶ is hydrogen, C₁-C₁₉-alkyl, C₇-C₁₀-phenylalkyl where the phenyl nucleus may be monosubstituted, disubstituted or trisubstituted by C₁-C₈-alkyl, chlorine, bromine, fluorine, hydroxyl, C₁-C₄-alkoxy, phenyl, phenoxy or carboxyl and the substituents can be identical or different, phenoxy C₁-C₄-alkyl, unsubstituted or C₁-C₈-alkyl-monosubstituted, -disubstituted or -trisubstituted C₃-C₁₀-cycloalkyl, unsubstituted or C₁-C₈-alkyl-, chlorine-, bromine-, fluorine-, hydroxyl-, C₁-C₄-alkoxy-, phenyl-, phenoxy- or carboxyl-monosubstituted, -disubstituted or -trisubstituted phenyl or naphthyl, a 5- or 6-membered saturated or aromatic O-, S- or N-containing heterocycle which may be monosubstituted, disubstituted, trisubstituted or tetrasubstituted by C₁-C₄-alkyl, or C₁-C₁₈-alkoxy where the alkyl chain may be interrupted by -O-, or phenoxy, naphthoxy, unsubstituted or C₁-C₄-alkyl-monosubstituted or - disubstituted C₅- or C₆-cycloalkoxy or phenylalkoxy of from 7 to 12 carbon atoms in total,
b) -CO-NH-R⁷ where R⁷ is hydrogen, C₁-C₁₈-alkyl, C₅-or C₆-cycloalkyl, phenyl which may be monosubstituted, disubstituted or trisubstituted by C₁-C₈-alkyl, chlorine, bromine, fluorine or C₁-C₄-alkoxy, or phenyl-C₁-C₄-alkyl, or
c) -SO₂-R⁸ where R⁸ is C₁-C₁₈-alkyl or unsubstituted or C₁-C₄-alkyl- or chlorine-substituted phenyl, or 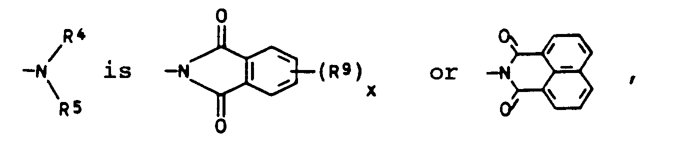 where R⁹ is C₁-C₈-alkyl, chlorine, bromine or fluorine and x is 0, 1, 2, 3 or 4, or - when n is 2 -
R⁴ is hydrogen and
R⁵ is 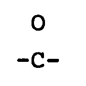 or 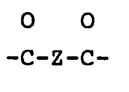 where
Z is a chemical bond, C₁-C₁₂-alkylene, C₄-C₈-oxaalkylene, phenylene, cyclohexylene, biphenylene, biphenylene oxide, dioxyphenylene, diaminophenylene, diaminocyclohexylene, 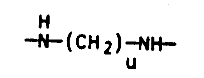 where u is from 2 to 6, -O-X-O-, where X is C₂-C₈-alkylene or C₄-C₈-oxaalkylene or a bivalent saturated or unsaturated 5- or 6-membered O-, S-or N-containing heterocycle, or 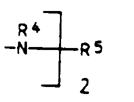 is a pyromellimide group,
or - when n is 3 -
R⁴ is hydrogen and
R⁵ is 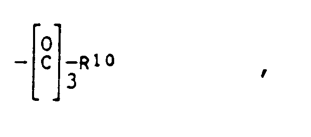 where
R¹⁰ is trivalent C₁-C₈-alkyl or trivalent phenyl,
or an acid addition salt or hydrate thereof.

2. A compound as claimed in claim 1, wherein R³ is hydrogen.

3. The use of a compound as claimed in claim 1 or 2 for stabilizing an organic material, in particular a plastics material.

4. The use of a compound as claimed in claim 1 or 2 for stabilizing a polyurethane.

5. The use of a compound as claimed in claim 1 or 2 for stabilizing a polyolefin.

6. The use of a compound as claimed in claim 1 or 2 for stabilizing a polyamide.

7. The use of a compound as claimed in claim 1 or 2 for stabilizing a paint.

8. The use of a compound as claimed in claim 1 or 2 as a metal ion deactivator.

9. A stabilized organic material, containing a compound as claimed in claim 1 or 2.

## Revendications

1. Composés de la formule générale (I) : 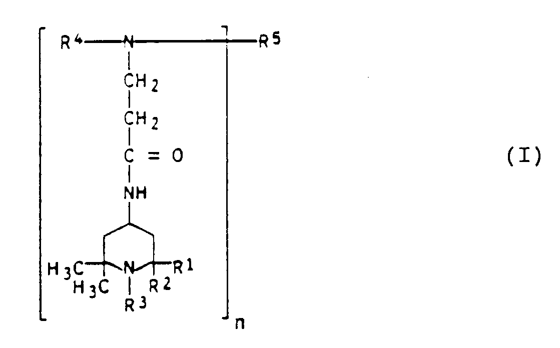 dans laquelle
R¹ et R² représentent des radicaux méthyle,
R³ représente un atome d'hydrogène, un radical alkyle en C₁-C₈, alcoxy(C₁-C₈)carbonyle, alcanoyle en C₁-C₈, benzyle, hydroxyéthyle, cyanométhyle ou aminoéthyle,
et
n est égal à 1, 2 ou 3, et -
lorsque n = 1 - alors
R⁴ représente un atome d'hydrogène, et
R⁵ représente
a) un radical 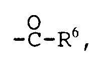 où R⁶ représente un atome d'hydrogène, un radical alkyle en C₁-C₁₉, phényl(C₇-C₁₀)alkyle, où le noyau phényle peut éventuellement être substitué par un, deux ou trois radicaux alkyle en C₁-C₈, atomes de chlore, de brome, de fluor, radicaux hydroxyle, alcoxy en C₁-C₄, phényle, phénoxy ou carboxyle et les substituants peuvent être identiques ou différents, un radical phénoxyalkyl(C₁-C₄), cycloalkyle en C₃-C₁₀ éventuellement une à trois fois substitué par des radicaux alkyle en C₁-C₈; un radical naphtyle ou phényle éventuellement substitué par des radicaux alkyle en C₁-C₈, des atomes de chlore, de brome, de fluor, des radicaux hydroxyle, alcoxy en C₁-C₄, phényle, phénoxy ou carboxyle et où le nombre des substituants peut s'élever jusqu'à 3; un noyau hétérocyclique pentagonal ou hexagonal, saturé ou aromatique, contenant O, S ou N, éventuellement substitué par 1 à 4 radicaux alkyle en C₁-C₄; un radical alcoxy en C₁-C₁₈, où la chaîne alkylique peut être interrompue par -O-; un radical phénoxy, un radical naphtoxy, un radical phénylalcoxy avec au total 7 à 12 atomes de carbone, ou un radical cycloalcoxy en C₅ ou C₆ une ou deux fois substitué par des radicaux alkyle en C₁-C₄,
b) un radical -CO-NH-R⁷, où R⁷ représente un atome d'hydrogène, un radical alkyle en C₁-C₁₈, un radical cycloalkyle en C₅ ou C₆, un radical phényle éventuellement substitué une à trois fois par des radicaux alkyle en C₁-C₈, des atomes de chlore, de brome, de fluor ou des radicaux alcoxy en C₁-C₄, ou un radical phénylalkyl(C₁-C₄), ou
c) un radical -SO₂-R⁸, où R⁸ représente un radical alkyle en C₁-C₁₈, ou un radical phényle substitué par des atomes de chlore ou des radicaux alkyle en C₁-C₄, ou bien 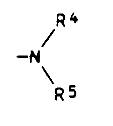
représente un radical 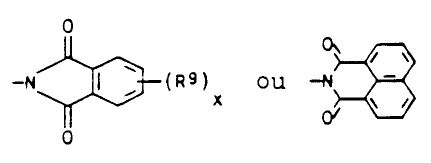 où
R⁹ représente un radical alkyle en C₁-C₈, un atome de chlore, de brome ou de fluor et x = 0, 1, 2, 3 ou 4,
ou - lorsque n = 2 - alors
R⁴ représente un atome d'hydrogene, et
R⁵ représente un groupe 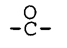 ou 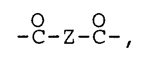 où
Z représente une liaison chimique, un radical alkylène en C₁-C₁₂, oxaalkylène en C₄-C₈, phénylène, cyclohexylène, diphénylène, oxyde de diphénylène, dioxyphénylène, diaminophénylène, diaminocyclohexylène, 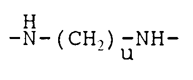 avec u=2 à 6, -O-X-O-, où X représente un radical alkylène en C₂-C₈ ou oxaalkylène en C₄-C₈, ou un noyau hétérocyclique divalent, saturé ou insaturé, pentagonal ou hexagonal, contenant O, S ou N, ou 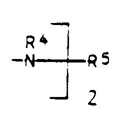
représente un groupe diimide d'acide pyromellitique, ou - lorsque n = 3 - alors
R⁴ représente un atome d'hydrogène, et
R⁵ représente un groupe 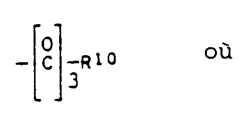
R¹⁰ représente un radical alkyle en C₁-C₈ trivalent, ou un radical phényle trivalent,
ainsi que les sels d'addition d'acides et les hydrates des composés (I).

2. Composé suivant la revendication 1, caractérisé en ce que R³ représente un atome d'hydrogène.

3. Utilisation des composés suivant la revendication 1 ou 2 en vue de la stabilisation d'une matière organique, plus particulièrement des matières plastiques.

4. Utilisation des composés suivant la revendication 1 ou 2 pour la stabilisation des polyuréthannes.

5. Utilisation des composés suivant la revendication 1 ou 2 pour la stabilisation des polyoléfines.

6. Utilisation des composés suivant la revendication 1 ou 2 pour la stabilisation des polyamides.

7. Utilisation des composés suivant la revendication 1 ou 2 pour la stabilisation des peintures.

8. Utilisation des composés suivant la revendication 1 ou 2 à titre de désactivateurs d'ions de métaux.

9. Matière organique stabilisée, contenant des composés suivant la revendication 1 ou 2.
